(19) **Europäisches Patentamt**
**European Patent Office**
**Office européen des brevets**

(11) **EP 3 641 550 B1**

(12) **EUROPEAN PATENT SPECIFICATION**

(45) Date of publication and mention of the grant of the patent:
**05.05.2021 Bulletin 2021/18**

(51) Int Cl.:
*A21D 2/26* *(2006.01)*     *A21D 8/04* *(2006.01)*
*A21D 10/00* *(2006.01)*   *A21D 13/066* *(2017.01)*
*C12N 9/10* *(2006.01)*    *A23L 29/00* *(2016.01)*
*A21D 13/40* *(2017.01)*   *A21D 13/43* *(2017.01)*
*A21D 13/42* *(2017.01)*   *A21D 13/41* *(2017.01)*
*A21D 13/16* *(2017.01)*

(21) Application number: **18731110.5**

(22) Date of filing: **20.06.2018**

(86) International application number:
**PCT/EP2018/066427**

(87) International publication number:
**WO 2018/234382 (27.12.2018 Gazette 2018/52)**

(54) **METHOD FOR IMPROVING DOUGH EXTENSIBILITY USING GAMMA GLUTAMYL TRANSPEPTIDASE**

VERFAHREN ZUR VERBESSERUNG DER DEHNBARKEIT VON TEIG UNTER VERWENDUNG VON GAMMA-GLUTAMYLTRANSPEPTIDASE

PROCEDE POUR AMELIORER L'EXTENSIBILITE DE LA PATE A L'AIDE DE LA GAMMA GLUTAMYL TRANSPEPTIDASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2017 EP 17177340**

(43) Date of publication of application:
**29.04.2020 Bulletin 2020/18**

(73) Proprietor: **Novozymes A/S**
**2880 Bagsvaerd (DK)**

(72) Inventors:
• **KALUM, Lisbeth**
**2880 Bagsvaerd (DK)**
• **LANDVIK, Sara, Maria**
**2880 Bagsvaerd (DK)**
• **MATVEEVA, Irina, Victorovna**
**Moscow 119330 (RU)**
• **JOERGENSEN, Steen, Troels**
**2880 Bagsvaerd (DK)**
• **JENSEN, Kenneth**
**2880 Bagsvaerd (DK)**

(74) Representative: **NZ EPO Representatives**
**Krogshoejvej 36**
**2880 Bagsvaerd (DK)**

(56) References cited:
**EP-A2- 0 441 353**      **WO-A1-2013/092731**
**DE-A1-102004 032 995**   **US-A- 4 645 672**

SEQUENCE LISTING

<110> Novozymes A/S

<120> DOUGH RELAXATION USING GAMMA GLUTAMYL TRANSPEPTIDASE

<130> 14491-WO-PCT

<160> 4

<170> Patent In version 3.5

<210> 1
<211> 585
<212> PRT
<213> Bacillus licheniformis

<400> 1

```
Met Arg Arg Leu Ala Phe Leu Val Val Ala Phe Cys Leu Ala Val Gly
1               5               10              15

Cys Phe Phe Ser Pro Val Ser Lys Ala Glu Gly Val Met Ser Gly Gly
            20              25              30

Gly Gly Asp Lys Val Ala Val Gly Lys Asp Gly Met Val Ala Thr Ala
        35              40              45

His Pro Leu Ala Ser Lys Ile Gly Ala Glu Val Leu Lys Lys Gly Gly
    50              55              60

Asn Ala Ile Asp Ala Ala Ile Ala Ile Gln Tyr Ala Leu Asn Val Thr
65              70              75              80

Glu Pro Met Met Ser Gly Ile Gly Gly Gly Phe Met Met Val Tyr
            85              90              95

Asp Gly Glu Thr Lys Glu Thr Ser Ile Ile Asn Ser Arg Glu Arg Ala
        100             105             110

Pro Glu Gly Ala Lys Pro Asp Met Phe Leu Asp Gly Asp Gly Lys Val
        115             120             125

Ile Pro Phe Ser Glu Arg Ser Arg His Gly Asn Ala Val Gly Val Pro
    130             135             140

Gly Thr Leu Lys Gly Leu Glu Ala Ala His Lys Lys Trp Gly Thr Lys
145             150             155             160

Lys Met Glu Asp Leu Ile Ser Pro Ser Ile Lys Leu Ala Glu Glu Gly
            165             170             175
```

```
Phe Pro Ile Asp Ser Val Leu Ala Asp Ala Ile Lys Asp His Gln Asp
            180                 185                 190

Lys Leu Ser Lys Thr Ala Ala Lys Asp Ile Phe Leu Pro Asp Gly Glu
            195                 200                 205

Pro Leu Lys Glu Gly Asp Ile Leu Val Gln Lys Asp Leu Ala Lys Thr
    210                 215                 220

Phe Lys Leu Ile Arg Lys Glu Gly Ser Lys Ala Phe Tyr Asp Gly Glu
225                 230                 235                 240

Ile Gly Arg Ala Ile Ala Asp Val Val Gln Asp Phe Gly Gly Ser Met
            245                 250                 255

Thr Pro Asp Asp Leu Ser Arg Tyr Glu Val Thr Thr Asp Lys Pro Ile
            260                 265                 270

Trp Gly Glu Tyr His Gly Tyr Asp Ile Ala Ser Met Pro Pro Pro Ser
            275                 280                 285

Ser Gly Gly Val Phe Met Leu Gln Met Leu Lys Leu Ile Asp Asp Phe
    290                 295                 300

His Leu Ser Gln Tyr Asp Pro Lys Ser Phe Glu Lys Tyr His Leu Leu
305                 310                 315                 320

Ala Glu Thr Met His Leu Ser Tyr Ala Asp Arg Ala Ala Tyr Ala Gly
            325                 330                 335

Asp Pro Glu Phe Val Asp Val Pro Leu Arg Gly Leu Leu Asp Pro Asp
            340                 345                 350

Tyr Ile Lys Glu Arg Gln Lys Leu Ile Ser Leu Asp Ser Met Asn Arg
            355                 360                 365

Asp Val Lys Glu Gly Asp Pro Trp Lys Tyr Glu Glu Gly Glu Pro Asn
    370                 375                 380

Tyr Glu Ile Val Pro Gln Pro Glu Asp Lys Thr Ile Gly Glu Thr Thr
385                 390                 395                 400

His Phe Thr Val Thr Asp Gln Trp Gly Asn Val Val Ser Tyr Thr Thr
            405                 410                 415

Thr Ile Glu Gln Leu Phe Gly Thr Gly Ile Leu Val Pro Gly Tyr Gly
            420                 425                 430
```

```
Leu Phe Leu Asn Asn Glu Leu Thr Asp Phe Asp Ala Val Pro Gly Gly
        435             440             445

Ala Asn Glu Val Gln Pro Asn Lys Arg Pro Leu Ser Ser Met Thr Pro
        450             455             460

Thr Ile Val Phe Lys Asp Glu Lys Pro Val Leu Thr Val Gly Ser Pro
465             470             475             480

Gly Gly Thr Thr Ile Ile Ala Ser Val Phe Gln Thr Ile Leu Asn Tyr
                485             490             495

Phe Glu Tyr Gly Met Ser Leu Gln Asp Ala Ile Glu Glu Pro Arg Ile
            500             505             510

Tyr Thr Asn Ser Leu Thr Ser Tyr Arg Tyr Glu Ser Gly Met Pro Glu
        515             520             525

Asp Val Arg Arg Lys Leu Asn Asp Phe Gly His Lys Phe Gly Ala Asn
        530             535             540

Pro Val Asp Ile Gly Asn Val Gln Ser Ile Phe Ile Asp Arg Glu Asn
545             550             555             560

Lys Thr Phe Met Gly Val Ala Asp Ser Ser Arg Asn Gly Thr Ala Val
            565             570             575

Gly Val Asn Ile Lys Thr Ser Ala Lys
            580             585
```

<210> 2
<211> 558
<212> PRT
<213> Bacillus horikoshii

<400> 2

```
Gln Lys Pro Val Lys Gly Ser Asn Glu Val Ala Val Gly Lys Asp Gly
1               5               10              15

Met Val Ser Thr Ser His Pro Leu Ala Ser Glu Ile Gly Ala Asp Ile
        20              25              30

Leu Arg Lys Gly Gly Asn Ala Met Asp Ala Ala Ile Ala Val Gln Phe
        35              40              45

Ala Leu Asn Val Val Glu Pro Met Met Ser Gly Ile Gly Gly Gly Gly
    50              55              60
```

Phe Met Met Val Tyr Asp Ala Glu Thr Asp Glu Thr Thr Ile Val Asn
65                  70              75                  80

Ser Arg Glu Arg Ala Pro Ala Gly Ala Thr Pro Asp Met Phe Leu Asn
                85              90                  95

Pro Asp Gly Ser Leu Ile Pro Phe Gln Glu Arg Val Arg His Gly Asn
            100             105             110

Ser Val Gly Val Pro Gly Thr Leu Lys Gly Leu Glu Ala Ala His Glu
            115             120             125

Lys Trp Gly Thr Arg Pro Phe Gln Gln Leu Ile Thr Pro Ala Phe Gln
            130             135             140

Leu Ala Gln Asn Gly Phe Ser Val Asp Arg Gln Leu Ala Leu Gln Ile
145             150             155             160

Glu Asn Asn Lys Glu Lys Leu Ala Gly Thr Ala Ala Lys Glu Val Phe
                165             170             175

Leu Pro Lys Gly Glu Pro Ile Lys Glu Gly Asp Trp Leu Val Gln Lys
            180             185             190

Asp Leu Ala Lys Thr Phe Lys Leu Ile Arg Ser His Gly Ser Glu Val
            195             200             205

Phe Tyr Asp Gly Glu Ile Gly Glu Ala Leu Ala Ala Thr Val Gln Asp
    210             215             220

Phe Gly Gly Ser Met Thr Ile Glu Asp Leu Gln Asn Tyr Gly Val Thr
225             230             235             240

Glu Asp Glu Pro Val Trp Gly Glu Tyr Lys Gly Tyr Asp Ile Ala Ser
            245             250             255

Met Pro Pro Pro Ser Ser Gly Gly Leu Phe Leu Leu Gln Met Leu Lys
            260             265             270

Thr Leu Asp Ser Phe Asp Ile Ser Gln Tyr Asp Arg Arg Ser Lys Glu
            275             280             285

Val Tyr His Leu Leu Ala Glu Ala Met His Leu Ser Tyr Ala Asp Arg
    290             295             300

Gly Ala Tyr Ala Gly Asp Pro Glu Phe Val Glu Val Pro Met Ile Gly
305             310             315             320

```
Leu Leu His Pro Asp Tyr Ile Ala Glu Arg Ser Ala Leu Ile Asp Ile
            325             330             335

Asn Ser Val Asn Thr Asn Pro Gln Pro Gly Asp Pro Trp Gln Tyr Glu
            340             345             350

Asp Val Asp Pro Asn Tyr Asn Val Ile Lys Gln Asn Asp Glu Lys Asp
            355             360             365

Ile Gly Glu Thr Thr His Phe Thr Val Ala Asp Arg Trp Gly Asn Leu
    370             375             380

Val Ser Tyr Thr Thr Thr Ile Glu Gln Val Phe Gly Ser Gly Ile Met
385             390             395             400

Val Pro Gly Tyr Gly Phe Met Leu Asn Asn Glu Leu Thr Asp Phe Asp
            405             410             415

Ala Arg Pro Gly Gly Ala Asn Glu Val Gln Pro Asn Lys Arg Pro Leu
            420             425             430

Ser Ser Met Thr Pro Thr Ile Val Phe Glu Asp Gly Lys Pro Ile Met
            435             440             445

Ser Val Gly Ser Pro Gly Gly Pro Thr Ile Ile Thr Ser Val Leu Gln
    450             455             460

Val Val Leu Asn Val Met Asp Tyr Glu Met Gly Leu Glu Glu Ala Ile
465             470             475             480

Ala Glu Pro Arg Ile Tyr Thr Asn Thr Ile Asn Ser Tyr Arg Tyr Glu
            485             490             495

Asp Gly Ile Ser Ala Glu Val Leu Ser Glu Leu Asn Ala Met Gly His
            500             505             510

Arg Phe Pro Ser Asn Ser Glu Leu Ile Gly Asn Val Gln Ser Ile Leu
            515             520             525

Ile Asp Tyr Glu Lys Asp Glu Tyr Val Gly Val Ala Asp Ala Arg Arg
    530             535             540

Asp Gly Ala Ser Val Gly Tyr Thr Arg Pro Gly Lys Arg Lys
545             550             555
```

<210> 3
<211> 61

<212> DNA
<213> Artificial sequence

<220>
<223> Primer

<400> 3

```
gactgaattc gagctctata aaaatgagga gggaaccgaa tgagacggtt agctttctta      60

g                                                                      61
```

<210> 4
<211> 41
<212> DNA
<213> Artificial sequence

<220>
<223> Primer

<400> 4
gactggatcc acgcgttact atttagccga tgtcttaatg t 41

**Description**

**Reference to a Sequence Listing**

This application contains a Sequence Listing in computer readable form.

**FIELD OF THE INVENTION**

The present invention relates to a process for improving the extensibility of dough, e.g., flattened dough, when producing, e.g., bread, flat bread, crackers, pizzas, pasta, noodles, laminated baking products, biscuits, baguettes, and hamburgers.

**BACKGROUND OF THE INVENTION**

Today, in the industrial dough-making processes, it is known to add dough-improving additives to the dough in order to improve parameters such as texture, volume, extensibility, and machine ability of the dough.
Reducing agents such as gluthathione, cysteine, malt, protease, sorbic acid, and non-leavening yeast are known dough-improving additives used to improve the extensibility of the dough.
WO2013/092731 discloses the application of glutamyl endopeptidases (EC 3.4.21.19) for increasing the extensional flow of dough during handling and proof.
There is still a need for finding improved extensibility solutions in the dough production, without, or only little effect, on other dough parameters, when making products such as bread, flat bread, crackers, pizzas, pasta, noodles, laminated baking products, biscuits, baguettes, and hamburgers.

**SUMMARY OF THE INVENTION**

Surprisingly, the inventors have found that gamma glutamyl transpeptidase (E.C. 2.3.2.2) increases the extensibility of a dough without, or very little, effect on other dough parameters, so we claim:
A method for increasing the extensibility of a dough comprising

    a) adding a gamma glutamyl transpeptidase (E.C.2.3.2.2) to flour or to a dough comprising a flour, wherein the gamma glutamyl transpeptidase has at least 60% identity with SEQ ID NO:1; and
    b) making the dough,

wherein the dough has an extensibility which is increased in comparison to the extensibility of a dough which is prepared under the same conditions, but without treatment with the gamma glutamyl transpeptidase.
In one embodiment, a flattened dough is produced from the dough.
In one embodiment, the dough is made into an edible product selected from the group consisting of bread, flat bread, crackers, pizzas, pasta, noodles, laminated baking products, biscuits, baguettes, and hamburgers.
In one embodiment, the flour is selected from the group consisting of wheat flour, corn flour, rye flour, barley flour, oat flour, rice flour, sorghum flour, and a combination thereof. The gamma glutamyl transpeptidase is a bacterial gamma glutamyl transpeptidase, in particular a *Bacillus* gamma glutamyl transpeptidase having at least 60% identity with SEQ ID NO:1.
In one embodiment, the gamma glutamyl transpeptidase is added in an amount of 0.01-100 mg of enzyme protein per kg of flour.
In one embodiment, additionally glutathione is added to the dough. The dough has an extensibility which is increased compared to the extensibility of a dough which is prepared under the same conditions, but without treatment with a gamma glutamyl transpeptidase.
In one embodiment, the dough further comprises one or more enzymes selected from the group consisting of amylase, maltogenic amylase, beta amylase, aminopeptidase, carboxypeptidase, catalase, cellulytic enzyme, chitinase, cutinase, cyclodextrin glycosyltransferase, deoxyribonuclease, esterase, glucan 1,4-alpha-maltotetrahydrolase, glucanase, galactanase, alpha-galactosidase, beta-galactosidase, glucoamylase, glucose oxidase, alpha-glucosidase, beta-glucosidase, haloperoxidase, hemicellulytic enzyme, invertase, laccase, lipase, mannanase, mannosidase, oxidase, pectinolytic enzymes, peptidoglutaminase, peroxidase, phospholipase, phytase, polyphenoloxidase, proteolytic enzyme, ribonuclease, transglutaminase, and xylanase.
In one embodiment, the flat bread is selected from the group consisting of tortillas, pita, Arabic bread, and Indian flat bread, including wheat and gluten free flat bread.
A premix comprising gamma glutamyl transpeptidase (E.C. 2.3.2.2) and flour is disclosed (this aspect not being according to the invention and present for illustration purpose only).

Said premix (which is not according to the invention and present for illustration purposes only) may further comprise one or more enzymes selected from the group consisting of amylase, maltogenic amylase, beta amylase, aminopeptidase, carboxypeptidase, catalase, cellulytic enzyme, chitinase, cutinase, cyclodextrin glycosyltransferase, deoxyribonuclease, esterase, glucanase, galactanase, alpha-galactosidase, beta-galactosidase, glucoamylase, glucose oxidase, alpha-glucosidase, beta-glucosidase, haloperoxidase, hemicellulytic enzyme, invertase, laccase, lipase, mannanase, mannosidase, oxidase, pectinolytic enzymes, peptidoglutaminase, peroxidase, phospholipase, phytase, polyphenoloxidase, proteolytic enzyme, ribonuclease, transglutaminase, and xylanase.

Additionally, the use of a gamma glutamyl transpeptidase (E.C.2.3.2.2) for increasing the extensibility of a dough is claimed, wherein the gamma glutamyl transpeptidase has at least 60% identity with SEQ ID NO: 1, wherein the dough has an extensibility which is increased compared to the extensibility of a dough which is prepared under the same conditions, but without treatment with the gamma glutamyl transpeptidase.

Disclosed is further a composition (which is not according to the invention and present for illustration purposes only) Z comprising a gamma glutamyl transpeptidase (E.C. 2.3.2.2), wherein the gamma glutamyl transpeptidase has at least 60% identity with SEQ ID NO:1.

Any composition, be it a premix, a dough, or a product prepared from the dough is not according to the invention and present for illustrative purposes only.

## DETAILED DESCRIPTION OF THE INVENTION

### Definitions

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$\text{(Identical Residues x 100)/(Length of Alignment – Total Number of Gaps in Alignment)}$$

**Variant:** The term "variant" means a polypeptide having gamma glutamyl transpeptidase activity comprising an alteration, *i.e.,* a substitution, insertion, and/or deletion, at one or more (e.g., several) positions. A substitution means replacement of the amino acid occupying a position with a different amino acid; a deletion means removal of the amino acid occupying a position; and an insertion means adding one or more amino acids adjacent to and immediately following the amino acid occupying a position.

**Improved property:** When the gamma glutamyl transpeptidase, according to the invention, is incorporated into a flour and/or a dough in effective amounts, one or more properties are improved compared to a flour and/or a dough in which the enzyme is not added.

The improved property is determined by comparison of a dough and/or a baked product prepared with and without addition of the enzyme of the present invention in accordance with the methods described below.

Organoleptic qualities may be evaluated using procedures well established in the baking industry, and may include, for example, the use of a panel of trained taste-testers.

**Improved extensibility:** The term "improved extensibility of the dough" is defined herein as the property of dough that can be subjected to increased stretching without rupture.

The increased stretching is a very important parameter as it means that it is possible to, e.g., obtain very thin doughs.

**Increased strength:** The term "increased strength of the dough" is defined herein as the property of dough that has generally more elastic properties and/or requires more work input to mould and shape.

**Increased elasticity:** The term "increased elasticity of the dough" is defined herein as the property of dough which has a higher tendency to regain its original shape after being subjected to a certain physical strain.

**Increased stability of the dough:** The term "increased stability of the dough" is defined herein as the property of dough that is less susceptible to mechanical abuse thus better maintaining its shape and volume and is evaluated by the ratio of height: width of a cross section of a loaf after normal and/or extended proof.

**Reduced stickiness of the dough:** The term "reduced stickiness of the dough" is defined herein as the property of a dough that has less tendency to adhere to surfaces, e.g., in the dough production machinery, and is either evaluated empirically by the skilled test baker or measured by use of a texture analyzer (e.g., TAXT2) as known in the art.

**Improved machine ability:** The term "improved machine ability of the dough" is defined herein as the property of a dough that is generally less sticky and/or more firm and/or more elastic.

**Increased volume of the dough/the baked product:** The term "increased volume of the dough/baked product" is measured as the volume of a dough or the volume of a given loaf of bread. The volume may, e.g., be determined by the rape seed displacement method, or by a skilled baker, or by using a Volscan profiler 600 as described in Example 2.

**Improved crumb structure of the baked product:** The term "improved crumb structure of the baked product" is defined herein as the property of a baked product regarding crumb uniformity, cell wall thickness, and the size of the individual gas cells pores on the slice of bread.

The crumb structure of the baked product is usually evaluated visually by the baker or by digital image analysis as known in the art (e.g., C-cell, Calibre Control International Ltd, Appleton, Warrington, UK).

**Improved softness of the baked product:** The term "improved softness of the baked product" is the opposite of "firmness" and is defined herein as the property of a baked product that is more easily compressed and is evaluated either empirically by the skilled test baker or measured by use of a texture analyzer (e.g., TAXT2 or TA-XT Plus from Stable Micro Systems Ltd, surrey, UK) as known in the art.

**Dough Compositions for making dough**

As used herein "dough" means any dough used to prepare a baked or cooked product.

The dough used to prepare a baked or cooked product may be made from any suitable dough ingredients comprising flour.

As used herein, a "flattened dough" means a dough, which typically has a thickness of one millimeter to a few centimeters.

The flattened dough may be used for making, e.g., flat bread, crackers, pizzas, pasta, noodles, laminated doughs, and biscuits.

A flat bread may be made from a simple mixture of flour, water, and salt and then thoroughly rolled into flattened dough. Flat bread has a very quick baking time (often <2 minutes).

The flat bread may be unleavened, i.e., made without a yeast, or leavened, e.g., made with a yeast.

The flat bread may include further optional ingredients, such as olive oil, sesame oil, shortenings, and spices.

Examples of flat bread include tortilla, pita, Arabic bread, and Indian flat bread, including wheat and gluten free flat bread.

Further non-limiting examples of flat bread include lavash, baladi, barbari, sangak, tandoor, taftoon, shami, halabi, mafrood, burr, bairuti, pocket bread, naan, phulka, chapatti, paratha, Arabic pita, Lebanese, mafrood, hapati, sangak, roti, taboon, shrak, mashrouh, nasir, tannoor, lavash, and taftan.

The dough used to prepare a flat bread product may be made from any suitable flour source, e.g., flour sourced from grains, such as, wheat flour, corn flour, rye flour, barley flour, oat flour, rice flour, or sorghum flour, potato flour, soy flour, flour from pulses, and combinations thereof.

Any flat bread process may be used to prepare the flat bread. The process of preparing flat bread generally involves the sequential steps of dough making (with an optional proofing step), sheeting or dividing, shaping and/or rolling, and proofing the dough, which steps are well known in the art.

The flattened dough may also be used to make pizzas. Pizza is a yeasted flatbread typically topped with, e.g., tomato sauce and cheese and baked in an oven.

The flattened dough may also be used to make crackers. A cracker is a baked food product made from a flattened dough. Flavorings or seasonings, such as salt, herbs, seeds, and/or cheese, may be added to the dough or sprinkled on top before baking as known in the art. Crackers come in many shapes and sizes - round, square, triangular, etc. Crackers are a kind of ancient flat bread.

The flattened dough according to the invention may also be used to make noodles and pasta.

Noodles are made from unleavened dough which is stretched, extruded, or rolled flat and cut into one of a variety of shapes. Noodles are usually cooked in boiling water, sometimes with cooking oil and/or salt added. They may be pan-fried or deep-fried.

Pasta is typically a noodle made from an unleavened dough of a durum wheat flour mixed with water and/or eggs and formed into sheets or various shapes, then cooked by boiling. Pasta can also be made with flour from other cereals or grains.

The flattened dough may also be used to make laminated baking products.

A laminated dough is a culinary preparation consisting of many thin layers of dough separated by butter, produced by repeated folding and rolling. Such doughs may contain many layers, i.e., more than 10 layers. During baking, the water in the butter vaporizes and expands, causing the dough to puff up and separate, while the lipids in the butter essentially fry the dough, resulting in a light, flaky product. Examples of laminated doughs include Croissant pastry, and other pastries such as Danish pastry, Flaky pastry, and Puff pastry.

The flattened dough may also be used to make biscuits.

The dough may be used to produce any baked or cooked product, in particular bread, flat bread, crackers, pizzas, pasta, noodles, laminated baking products, biscuits, baguettes, and hamburgers.

The dough may also comprise other conventional dough relaxation ingredients such as glutathion, protease, malt, sorbic acid, L-cysteine, and/or yeast extract.

There may be a synergistic effect between gamma glutamyl transpeptidase and glutathion.

There may be a synergistic effect between gamma glutamyl transpeptidase and protease.

There may be a synergistic effect between gamma glutamyl transpeptidase and malt.

There may be a synergistic effect between gamma glutamyl transpeptidase and sorbic acid.

There may be a synergistic effect between gamma glutamyl transpeptidase and L-cysteine.

There may be a synergistic effect between gamma glutamyl transpeptidase and yeast extract.

The dough may also comprise one or more emulsifiers. Emulsifiers also serve to improve dough extensibility. Examples of suitable emulsifiers are mono- or diglycerides, polyoxyethylene stearates, diacetyl tartaric acid esters of monoglycerides, sugar esters of fatty acids, propylene glycol esters of fatty acids, polyglycerol esters of fatty acids, lactic acid esters of monoglycerides, acetic acid esters of monoglycerides, lecithin or phospholipids, or ethoxylated monoglycerides. Particular emulsifiers include monoglycerides, diacetyl tartaric acid esters of monoglyceride (DATEM) and sodium stearoyl lactylate (SSL).

Other conventional ingredients that may be added to the dough include proteins, such as milk powder, gluten, and soy; eggs (either whole eggs, egg yolks or egg whites); an oxidant such as ascorbic acid, potassium bromate, potassium iodate, azodicarbonamide (ADA), ammonium persulfate or potassium persulfate; a sugar such as sucrose, dextrose, etc.; a salt such as sodium chloride, calcium acetate, sodium sulfate or calcium sulfate, diluents such silica dioxide, starch of different origins. Still other convention ingredients include hydrocolloids such as CMC, guar gum, xanthan gum, locust bean gum, etc. Modified starches may be also used.

The dough may be a fiber dough, e.g., the dough may contain grains, e.g., whole wheat, and/or are enriched with extra fibres in the form of, e.g., cereal bran, e.g., wheat bran. Wheat bran is produced as a side product of milling wheat into white flour.

Normally, fibres are divided into fine fibres, medium fibres, and coarse fibres as known in the art. Fine fibres are particularly useful in the present invention.

In addition to preparing fresh flattened dough or fresh flattened dough products, also a frozen flattened dough or a frozen flattened dough product can be prepared.

Flattened dough and products obtained from flattened dough may be prepared in industrialized processes, where the products are prepared mechanically using automated or semi-automated equipment.

**Enzymes**

Gamma Glutamyl transpeptidase

Gamma glutamyl transpeptidase (E.C. 2.3.2.2) plays a major role in glutathione metabolism where the enzyme catalyzes the transfer of the gamma glutamyl group from gamma glutamyl compounds to amino acids, peptide acceptors, or water (Tate and Meister, 1981, Mol. Cell. Biochem. 39: 357-368). For example, gamma glutamyl transpeptidase catalyzes the hydrolysis of glutathione to produce glutamic acid, and the transfer of the gamma-glutamyl group of glutathione to an amino acid.

Gamma glutamyl transpeptidases have been reported from, e.g., *Bacillus subtilis* (JP 4281787), *Bacillus natto* (JP 2065777), and *Bacillus agaradhaerens* (WO 02/077009).

According to the present invention, the gamma glutamyl transpeptidase is a bacterial gamma glutamyl transpeptidase; in particular a *Bacillus* gamma glutamyl transpeptidase; in particular a *Bacillus licheniformis* or a *Bacillus horikoshii* gamma glutamyl transpeptidase, wherein the gamma glutamyl transpeptidase is an enzyme having at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to the polypeptide of SEQ ID NO: 1.

In one embodiment, the gamma glutamyl transpeptidase is an enzyme having the amino acid sequence shown in SEQ ID NO:1 herein:

```
MRRLAFLVVA FCLAVGCFFS PVSKAEGVMS GGGGDKVAVG KDGMVATAHP LASKIGAEVL KKGGNAIDAA
IAIQYALNVT EPMMSGIGGG GFMMVYDGET KETSIINSRE RAPEGAKPDM FLDGDGKVIP FSERSRHGNA
VGVPGTLKGL EAAHKKWGTK KMEDLISPSI KLAEEGFPID SVLADAIKDH QDKLSKTAAK DIFLPDGEPL
KEGDILVQKD LAKTFKLIRK EGSKAFYDGE IGRAIADVVQ DFGGSMTPDD LSRYEVTTDK PIWGEYHGYD
IASMPPPSSG GVFMLQMLKL IDDFHLSQYD PKSFEKYHLL AETMHLSYAD RAAYAGDPEF VDVPLRGLLD
PDYIKERQKL ISLDSMNRDV KEGDPWKYEE GEPNYEIVPQ PEDKTIGETT HFTVTDQWGN VVSYTTTIEQ
LFGTGILVPG YGLFLNNELT DFDAVPGGAN EVQPNKRPLS SMTPTIVFKD EKPVLTVGSP GGTTIIASVF
QTILNYFEYG MSLQDAIEEP RIYTNSLTSY RYESGMPEDV RRKLNDFGHK FGANPVDIGN VQSIFIDREN
KTFMGVADSS RNGTAVGVNI KTSAK
```

In another embodiment, the gamma glutamyl transpeptidase is an enzyme having the amino acid sequence shown in SEQ ID NO:2 herein:

```
QKPVKGSNEVAVGKDGMVSTSHPLASEIGADILRKGGNAMDAAIAVQFALNVVEPMMSGIGGGGFMMVYDAETDETTIVNSRERA
PAGATPDMFLNPDGSLIPFQERVRHGNSVGVPGTLKGLEAAHEKWGTRPFQQLITPAFQLAQNGFSVDRQLALQIENNKEKLAGT
AAKEVFLPKGEPIKEGDWLVQKDLAKTFKLIRSHGSEVFYDGEIGEALAATVQDFGGSMTIEDLQNYGVTEDEPVWGEYKGYDIA
SMPPPSSGGLFLLQMLKTLDSFDISQYDRRSKEVYHLLAEAMHLSYADRGAYAGDPEFVEVPMIGLLHPDYIAERSALIDINSVN
TNPQPGDPWQYEDVDPNYNVIKQNDEKDIGETTHFTVADRWGNLVSYTTTIEQVFGSGIMVPGYGFMLNNELTDFDARPGGANEV
QPNKRPLSSMTPTIVFEDGKPIMSVGSPGGPTIITSVLQVVLNVMDYEMGLEEAIAEPRIYTNTINSYRYEDGISAEVLSELNAM
GHRFPSNSELIGNVQSILIDYEKDEYVGVADARRDGASVGYTRPGKRK
```

The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope, or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

A gamma glutamyl transpeptidase may typically be added in an effective amount such as in the range of 0.01-100 mg of enzyme protein per kg of flour, e.g., 0.1-50 mg of enzyme protein per kg of flour, e.g., 0.5-50 mg of enzyme protein per kg of flour, e.g., 1-50 mg of enzyme protein per kg of flour.

Additional enzymes

Optionally, one or more additional enzymes, such as amylase, maltogenic amylase, beta amylase, aminopeptidase, carboxypeptidase, catalase, cellulytic enzyme, chitinase, cutinase, cyclodextrin glycosyltransferase, deoxyribonuclease, esterase, glucan 1,4-alpha-maltotetrahydrolase, glucanase, galactanase, alpha-galactosidase, beta-galactosidase, glucoamylase, glucose oxidase, alpha-glucosidase, beta-glucosidase, haloperoxidase, hemicellulytic enzyme, invertase, laccase, lipase, mannanase, mannosidase, oxidase, pectinolytic enzymes, peptidoglutaminase, peroxidase, phospholipase, phytase, polyphenoloxidase, proteolytic enzyme, ribonuclease, transglutaminase, and xylanase may be used together with the enzyme composition employed in the invention.

The additional enzyme(s) may be of any origin, including mammalian, plant, and microbial (bacterial, yeast or fungal) origin.

Suitable commercial alpha-amylase compositions include, e.g., BAKEZYME P 300 (available from DSM) and FUNGAMYL 2500 BG, FUNGAMYL 4000 BG, FUNGAMYL 800 L, FUNGAMYL ULTRA BG and FUNGAMYL ULTRA SG (available from Novozymes A/S).

The maltogenic alpha-amylase (EC 3.2.1.133) may be from *Bacillus*. A maltogenic alpha-amylase from B. *stearothermophilus* strain NCIB 11837 is commercially available from Novozymes A/S under the tradename Novamyl®.

The maltogenic alpha-amylase may also be a variant of the maltogenic alpha-amylase from B. *stearothermophilus* as disclosed in, e.g., WO1999/043794; WO2006/032281; or WO2008/148845, e.g., Novamyl® 3D.

An anti-staling amylase for use in the invention may also be an amylase (glucan 1,4-alpha-maltotetrahydrolase (EC 3.2.1.60)) from *Pseudomonas saccharophilia* or variants thereof, such as any of the amylases disclosed in WO1999/050399, WO2004/111217 or WO2005/003339.

The glucoamylase for use in the present invention include the *A. niger* G1 or G2 glucoamylase (Boel et al. (1984), EMBO J. 3 (5), p. 1097-1102), the *A. awamori* glucoamylase disclosed in WO 84/02921, or the *A. oryzae* glucoamylase (Agric. Biol. Chem. (1991), 55 (4), p. 941-949). A suitable commercial glucoamylase is GoldCrust® obtainable from Novozymes A/S.

The glucose oxidase may be a fungal glucose oxidase, in particular an *Aspergillus niger* glucose oxidase (such as GLUZYME®, available from Novozymes A/S).

The xylanase which may be of microbial origin, e.g., derived from a bacterium orfungus, such as a strain of *Aspergillus,* in particular of *A. aculeatus, A. niger, A. awamori,* or *A. tubigensis,* from a strain of *Trichoderma,* e.g. *T. reesei,* or from a strain of *Humicola,* e.g., *H. insolens.*

Suitable commercially available xylanase preparations for use in the present invention include PANZEA BG, PENTOPAN MONO BG and PENTOPAN 500 BG (available from Novozymes A/S), GRINDAMYL POWERBAKE (available from Danisco), and BAKEZYME BXP 5000 and BAKEZYME BXP 5001 (available from DSM).

The protease may be from *Bacillus,* e.g., B. *amyloliquefaciens.* A suitable protease may be Nuetrase® available from Novozymes A/S.

The phospholipase may have phospholipase A1, A2, B, C, D or lysophospholipase activity; it may or may not have lipase activity. It may be of animal origin, e.g., from pancreas, snake venom or bee venom, or it may be of microbial origin, e.g., from filamentous fungi, yeast or bacteria, such as *Aspergillus or Fusarium, e.g., A. niger, A. oryzae* or *F. oxysporum.* A preferred lipase/phospholipase from *Fusarium oxysporum* is disclosed in WO 98/26057. Also, the variants described in WO 00/32758 may be used.

Suitable phospholipase compositions are LIPOPAN F and LIPOPAN XTRA (available from Novozymes A/S) or PANAMORE GOLDEN and PANAMORE SPRING (available from DSM).

## Enzyme Treatment

The gamma glutamyl transpeptidase according to the invention is added to the dough ingredients, e.g., indirectly to the dough by adding it to the flour used to prepare the dough, or directly to the dough itself.

The gamma glutamyl transpeptidase may be added to flour or dough in any suitable form, such as, e.g., in the form of a liquid, in particular a stabilized liquid, or it may be added to flour or dough as a substantially dry powder or granulate.

Granulates may be produced, e.g., as disclosed in US Patent No. 4,106,991 and US Patent No. 4,661,452. Liquid enzyme preparations may, for instance, be stabilized by adding a sugar or sugar alcohol or lactic acid according to established procedures. Other enzyme stabilizers are well-known in the art.

## Pre-Mixes

It will often be advantageous to provide the enzyme(s) used in the treatment of the present invention in admixture with other ingredients used to improve the properties of dough products. These are commonly known in the art as "premixes," which usually comprise flour.

Hence, premixes for improving the quality of dough used to prepare a flat bread product or flat bread products, which premix comprises gamma glutamyl transpeptidase and one or more dough ingredients, in particular flour such as flour from grains, such as, wheat flour, corn flour, rye flour, barley flour, oat flour, rice flour, or sorghum flour, and any combinations thereof are disclosed. (Said premixes are not according to the present invention and present for illustrative purposes only.)

The premix may also comprise one or more enzymes selected from the group consisting of amylase, maltogenic amylase, beta amylase, aminopeptidase, carboxypeptidase, catalase, cellulytic enzyme, chitinase, cutinase, cyclodextrin glycosyltransferase, deoxyribonuclease, esterase, glucan 1,4-alpha-maltotetrahydrolase, glucanase, galactanase, alpha-galactosidase, beta-galactosidase, glucoamylase, glucose oxidase, alpha-glucosidase, beta-glucosidase, haloperoxidase, hemicellulytic enzyme, invertase, laccase, lipase, mannanase, mannosidase, oxidase, pectinolytic enzymes, peptidoglutaminase, peroxidase, phospholipase, phytase, polyphenoloxidase, proteolytic enzyme, ribonuclease, transglutaminase, and xylanase.

The pre-mix composition may be in liquid form or dry or substantially dry form.

The present invention is further described by the following examples which should not be construed as limiting the scope of the invention.

**EXAMPLES**

**Example 1** (preparatory Example, enzyme itself not claimed in the present invention)

Gamma Glutamyl Transpeptidase (GGT) expression (SEQ ID NO:1)

The Gamma Glutamyl Transpeptidase (GGT) gene was identified in *Bacillus licheniformis* (ATCC PTA-7992) encoding the GGT protein, SEQ ID NO:1.

Two oligonucleotide primers were designed (SEQ ID NO:3 and SEQ ID NO:4), which allowed PCR amplification of the entire GGT open reading frame (ORF), with the ribosome binding site (RBS) from a *Bacillus clausii* alkaline protease gene inserted in front of the GGT signal peptide. The upstream primer, SEQ ID NO:3, incorporated EcoRI and SacI sites in front of the alkaline protease RBS preceding the GGT start.

The downstream primer, SEQ ID NO:4, incorporated MluI and BamHI following the GGT stop codon.

SEQ ID NO:3:

5'-
GACTGAATTCGAGCTCTATAAAAATGAGGAGGGAACCGAATGAGACGGTTAGCTTTCTTAG
-3'

SEQ ID NO:4:
5'-GACTGGATCCACGCGTTACTATTTAGCCGATGTCTTAATGT-3'

Chromosomal DNA from *B. licheniformis* PL1980 (US 8431382) was used as template in a PCR reaction with primers SEQ ID NO:3 and SEQ ID NO:4 in which the annealing temperature was ramped down from 62°C C to 52°C in steps of 1°C, then kept constant at 57°C for 20 cycles.

A PCR fragment of approximately 1.8 kb was obtained, digested with SacI + MluI, and cloned into the 3.3 kb SacI-MluI vector fragment from pSJ6814 (described in EP 1766002 B1).

The ligation mixture was transformed into an *E. coli* laboratory strain by electroporation, selecting ampicillin resistance, and a transformant with the correct DNA sequence of the PCR amplified segment was kept.

The 2.35 kb EcoRI-MluI segment containing the *cryIIIA*_stab-*ggt* construct was excised from the transformant, and ligated to the 4.75 kb MluI-EcoRI fragment of pSJ6869 (described in US 20140106457).
The ligation mixture was transformed into *B. subtilis* laboratory strain selecting erythromycin resistance (2 microgram/ml) at 30°C, and a correct transformant was kept.

The correct transformant was transformed into B. *subtilis* conjugative donor strain PP289-5 (US 6066473), resulting in a strain that was used as donor in conjugations to *B. licheniformis* host strain PP1897-3 (US 8431382). Tetracycline sensitive trans-conjugants were isolated, and colonies with a very weak or absent amylase phenotype were isolated following plasmid integration at 50°C, with ErmR selection. These integrants were propagated at 30°C, to allow plasmid replication and loss, and an amylase negative, erythromycin sensitive strain was obtained.
This *Bacillus licheniformis* strain was grown as known in the art, and the GGT (SEQ ID NO:1) was recovered as known in the art.

**Example 2**

Gamma glutamyl transpeptidase (SEQ ID NO:1) in baking

Bread was prepared using a straight dough procedure according to below recipe and process conditions. All chemicals applied were food grade. Fungamyl 2500 BG (2500 FAU/g) is available from Novozymes A/S.
Gamma glutamyl transpeptidase (GGT - SEQ ID NO:1) may be made as described in Example 1.

**Table 1: Dough Recipe**

| Ingredient | Amount (on flour basis) |
|---|---|
| Flour<br>80% Kolibri (Meneba, NL)<br>20% Victory Landmel N+ (HavneMøllerne, DK) | 100% |

(continued)

| Ingredient | Amount (on flour basis) |
|---|---|
| Tap water | 61% |
| Yeast (fresh) | 3.4% |
| Sucrose | 1.5% |
| Salt | 1.5% |
| Ascorbic acid | 24 ppm |
| Calcium Propionate | 0.3% |
| Fungamyl 2500 BG (Novozymes A/S) | 10 FAU/kg |
| Gamma glutamyl transpeptidase (SEQ ID No:1) | 0; 10; 20; 30; 40 ppm |

Procedure:

All ingredients were weighed out. Salt, sucrose, yeast, ascorbic acid, calcium propionate and enzyme were added to the mixing bowl. Tap water was weighed out, and the temperature adjusted with ice (to approx. 9-10°C, in order to reach a dough temperature of 27°C after mixing) and added to the mixing bowl. 2500 g flour (2000 g Kolibri and 500 g Victory) were added to the mixing bowl, and all ingredients were mixed for 3 min at 63 rpm and 7 min at 90 rpm using Spiral mixer (DIOSNA Dierks & Söhne GmbH, DE). The mixed dough was taken out of the mixing bowl and the temperature was controlled, and dough parameters were determined manually (as described in the section - Manual dough evaluation).

The dough was divided into pieces of 450 g each, rounded by hand, where after they rested for 15 min at room temperature covered by plastic. The rested dough pieces were shaped into bread in a sheeter (MO671 MPB-001, Glimek, SE) and transferred to greased 1400 ml pans (Top 230x115x68 mm). The bread was proofed at 32°C at 86% humidity for 60 min. The proofed bread was baked for 35 min in a deck oven (Piccolo, Wachtel, DE) at 225°C with steam. The bread was taken out of the pans and allowed to cool to room temperature. Volume of bread was determined as described under volume determination.

**Table 2: Manual dough evaluation**

| The dough properties were evaluated approx. 5 min after mixing. A scale between 0-10 was used and dough properties were evaluated relative to a control without addition of GGT. The control was given the value 5. Details regarding definition, evaluation and scale is found in below table. | | | |
|---|---|---|---|
| Parameter | Definition | Evaluation method | Scale |
| Stickiness | The degree to which a dough adheres to one's hands or other surfaces | A 3 cm deep cut was made in the middle of the dough. Stickiness was evaluated by touch of the fresh cut by the whole palm of a hand | Less sticky 0-4 Control 5 More sticky 6-10 |
| Softness | The degree to, or ease with, which a dough will compress or resist compression | Softness was measured by squeezing and feeling the dough by hand | Less soft 0-4 Control 5 More soft 6-10 |
| Elasticity | The ability of a dough to resist stretching as well as to return to its original size and shape when the force is removed | A piece of dough (approx. 30 g) was rolled to a dough string of 10 cm which was pulled gently in each end to feel the resistance and elasticity | Less elastic 0-4 Control 5 More elastic 6-10 |
| Extensibility | The degree to which a dough can be stretched without tearing | A piece of dough (approx. 30 g) was gently stretched to form a "window" to feel extensibility | Less extensible 0-4 Control 5 More extensible 6-10 |

Volume determination:

The specific volume was measured using the Volscan profiler 600 (Stable microsystems, UK) running on the Volscan profiler software. Each bread was mounted in the machine. The weight of each loaf was automatically determined with the build-in balance of the Volscan instrument. The volume of each loaf was calculated from a 3D image created by the instrument when each loaf of bread was rotated with a speed of 1.5 revolutions per second while it was scanned with a laser beam taking 3 mm vertical steps per revolution. Specific volume was calculated for each bread according to the following formula:

$$\text{Specific volume (ml/g)} = \text{volume (ml)/weight (g)}$$

The reported value was the average of 2 bread from the same dough.

**Table 3: Results**

|  | Control | 10 ppm GGT | 20 ppm GGT | 30 ppm GGT | 40 ppm GGT |
|---|---|---|---|---|---|
| **Dough stickiness** | 5 | 5 | 5 | 5 | 5 |
| **Dough softness** | 5 | 5 | 5 | 5 | 6 |
| **Dough extensibility** | 5 | 6 | 7 | 7 | 7 |
| **Dough elasticity** | 5 | 5 | 5 | 5 | 5 |
| **Bread Specific volume (ml/g)** | 4.40 | 4.35 | 4.46 | 4.47 | 4.42 |

Conclusion

Surprisingly, addition of GGT resulted in a significantly more extensible dough, while no effect on other dough properties was observed. No effect on bread volume was seen.

**Example** 3 (preparatory Example, enzyme itself not claimed in the present invention)

Gamma glutamyl transpeptidase (SEQ ID NO:2)

A Gamma Glutamyl Transpeptidase (GGT) gene was identified in a *Bacillus horikoshii* strain. The *Bacillus horikoshii* strain was found in New Zealand with a registration date of 15 May 1982. The *Bacillus horikoshii* Gamma Glutamyl Transpeptidase, SEQ ID NO:2, has the following mature protein sequence:

QKPVKGSNEVAVGKDGMVSTSHPLASEIGADILRKGGNAMDAAIAVQFALNVVEPMMSGIGGG
GFMMVYDAETDETTIVNSRERAPAGATPDMFLNPDGSLIPFQERVRHGNSVGVPGTLKGLEAA
HEKWGTRPFQQLITPAFQLAQNGFSVDRQLALQIENNKEKLAGTAAKEVFLPKGEPIKEGDWL
VQKDLAKTFKLIRSHGSEVFYDGEIGEALAATVQDFGGSMTIEDLQNYGVTEDEPVWGEYKGY
DIASMPPPSSGGLFLLQMLKTLDSFDISQYDRRSKEVYHLLAEAMHLSYADRGAYAGDPEFVE
VPMIGLLHPDYIAERSALIDINSVNTNPQPGDPWQYEDVDPNYNVIKQNDEKDIGETTHFTVADR
WGNLVSYTTTIEQVFGSGIMVPGYGFMLNNELTDFDARPGGANEVQPNKRPLSSMTPTIVFED
GKPIMSVGSPGGPTIITSVLQVVLNVMDYEMGLEEAIAEPRIYTNTINSYRYEDGISAEVLSELNA
MGHRFPSNSELIGNVQSILIDYEKDEYVGVADARRDGASVGYTRPGKRK

SEQ ID NO:2 was expressed as an extracellular protein in a *Bacillus subtilis* host strain as known in the art.
SEQ ID NO:2 showed 68% sequence identity to SEQ ID NO:1.

**Example 4**

Gamma glutamyl transpeptidases (SEQ ID NO:1 and SEQ ID NO:2) in baking

Doughs were prepared using a straight dough procedure according to below recipe and process conditions.

All chemicals applied were food grade. Gamma glutamyl transpeptidases (GGT) were added in concentrations as stated in Table 4.

Table 4: Dough Recipe:

| Ingredient | Amount (on flour basis) |
|---|---|
| Flour<br>80% Kolibri (Meneba, NL)<br>20% Victory Landmel N+ (HavneMøllerne, DK) | 100% |
| Tap water | 58% |
| Yeast (fresh) | 4% |
| Sucrose | 1.5% |
| Salt | 1.5% |
| Ascorbic acid | 30 ppm |
| Gamma glutamyl transpeptidase (SEQ ID No:1; SEQ ID No:2) | 0; 0.50; 2 mg enzyme protein/kg flour |

Procedure:

All ingredients were weighed out. A stock solution comprising salt, sucrose and ascorbic acid was prepared in tap water and stored on use ice until use. Further a stock solution of yeast was prepared in tap water. Tap water was weighed out; the temperature adjusted (in order to reach a dough temperature of 26°C after mixing) and then added to the mixing bowl.

100 g flour (80 g Kolibri and 20 g Victory), salt/sugar/ascorbic acid stock solution, GGT and yeast solution were added to the mixing bowl and mixed for 5 min using a 100 g mixer (National MFG Co, Nebraska, Model 100-200A). The mixed dough was taken out of the mixing bowl, rounded to form a ball shape, and the temperature was recorded. Dough parameters were determined manually by hand.

Manual dough evaluation:

The dough properties were evaluated approximately 2 min after mixing.

A scale between 0-10 was used, and dough properties were evaluated relative to a control without addition of GGT. The control was run in triplicates and given the value 5.

Softness and elasticity were evaluated first, then the dough ball was cut in half using a sharp knife. Stickiness was measured in the fresh cut. Extensibility was evaluated twice on each dough balls (2 half pieces). Further details regarding definition, evaluation, and scale are found in below Table 5.

Table 5: Dough evaluation parameters

| Parameter | Definition | Evaluation method | Scale |
|---|---|---|---|
| Softness | The degree to, or ease with, which a dough will compress or resist compression | Softness was measured by squeezing and feeling the dough by hand | Less soft 0-4<br>Control 5<br>More soft 6-10 |
| Elasticity | The ability of a dough to resist stretching as well as to return to its original size and shape when the force is removed | A small piece of dough was gently pulled from the dough ball using two fingers to feel the resistance and elasticity. This was done twice on each dough. | Less elastic 0-4<br>Control 5<br>More elastic 6-10 |

(continued)

| Parameter | Definition | Evaluation method | Scale |
|-----------|-----------|-------------------|-------|
| Stickiness | The degree to which a dough adheres to one's hands or other surfaces | The dough ball was cut in half. Stickiness was evaluated by touch of the fresh cut by the whole palm of a hand | Less sticky 0-4 Control 5 More sticky 6-10 |
| Extensibility | The degree to which a dough can be stretched without tearing | A piece of dough (approx. 80 g) was gently stretched to form a "window" to feel extensibility | Less extensible 0-4 Control 5 More extensible 6-10 |

Table 6: Results

| | Control | SEQ ID NO: 1 0.5 mg EP | SEQ ID NO: 1 2 mg EP | SEQ ID NO: 2 0.5 mg EP | SEQ ID NO: 2 2 mg EP |
|---|---|---|---|---|---|
| Dough stickiness | 5 | 5 | 5 | 4 | 4 |
| Dough softness | 5 | 5 | 5 | 5 | 5 |
| Dough extensibility | 5 | 6 | 6 | 6 | 7 |
| Dough elasticity | 5 | 5 | 5 | 5 | 5 |

Conclusion

Both SEQ ID No. 1 and SEQ ID No.2 showed a clear increase on dough extensibility while none or little effect on other dough properties were observed.

## Claims

1. A method for increasing the extensibility of a dough comprising

   a) adding a gamma glutamyl transpeptidase (E.C.2.3.2.2) to flour or to a dough comprising a flour, wherein the gamma glutamyl transpeptidase has at least 60% identity with SEQ ID NO:1; and
   b) making the dough,

   wherein the dough has an extensibility which is increased compared to the extensibility of a dough which is prepared under the same conditions, but without treatment with the gamma glutamyl transpeptidase.

2. The method according to claim 1, wherein a flattened dough is produced from the dough.

3. The method according to any of the preceding claims, wherein the dough is made into an edible product selected from the group consisting of bread, flat bread, crackers, pasta, noodles, laminated baking products, biscuits, baguettes, hamburgers, and pizzas.

4. The method according to any of the preceding claims, wherein the flour is selected from the group consisting of wheat flour, corn flour, rye flour, barley flour, oat flour, rice flour, sorghum flour, and a combination thereof.

5. The method according to any of the preceding claims, wherein the gamma glutamyl transpeptidase is added in an amount of in the range of 0.01-100 mg of enzyme protein per kg of flour.

6. The method according to any of the preceding claims, wherein additionally glutathione is added.

7. The method according to any of the preceding claims, wherein the dough further comprises one or more enzymes selected from the group consisting of amylase, maltogenic amylase, beta amylase, aminopeptidase, carboxypeptidase, catalase, cellulytic enzyme, chitinase, cutinase, cyclodextrin glycosyltransferase, deoxyribonuclease, esterase, glucan 1,4-alpha-maltotetrahydrolase, glucanase, galactanase, alpha-galactosidase, beta-galactosidase, glucoamylase, glucose oxidase, alpha-glucosidase, beta-glucosidase, haloperoxidase, hemicellulytic enzyme, invertase, laccase, lipase, mannanase, mannosidase, oxidase, pectinolytic enzymes, peptidoglutaminase, peroxidase, phospholipase, phytase, polyphenoloxidase, proteolytic enzyme, ribonuclease, transglutaminase, and xylanase.

**8.** The method according to any of the preceding claims, wherein the flat bread are selected from the group consisting of tortillas, pita, Arabic bread, Indian flat bread, wheat and gluten free flat bread.

**9.** Use of a gamma glutamyl transpeptidase (E.C.2.3.2.2) for increasing the extensibility of a dough, wherein the gamma glutamyl transpeptidase has at least 60% identity with SEQ ID NO:1, wherein the dough has an extensibility which is increased compared to the extensibility of a dough which is prepared under the same conditions, but without treatment with the gamma glutamyl transpeptidase.

**Patentansprüche**

**1.** Verfahren zur Erhöhung der Dehnbarkeit eines Teiges, umfassend

a) Zugabe einer Gamma-Glutamyltranspeptidase (E.C.2.3.2.2) zu Mehl oder zu einem Teig, der ein Mehl umfasst, wobei die Gamma-Glutamyltranspeptidase mindestens 60 % Identität mit SEQ ID NO:1 aufweist; und
b) Herstellen des Teigs,

wobei der Teig eine Dehnbarkeit aufweist, die im Vergleich zu der Dehnbarkeit eines Teigs, der unter den gleichen Bedingungen, aber ohne Behandlung mit der Gamma-Glutamyltranspeptidase hergestellt wird, erhöht ist.

**2.** Verfahren nach Anspruch 1, wobei aus dem Teig ein Teigfladen hergestellt wird.

**3.** Verfahren nach einem der vorhergehenden Ansprüche, wobei aus dem Teig ein essbares Produkt hergestellt wird, das ausgewählt ist aus der Gruppe bestehend aus Brot, Fladenbrot, Crackern, Teigwaren, Nudeln, laminierten Backprodukten, Keksen, Baguettes, Hamburgern und Pizzen.

**4.** Verfahren nach einem der vorhergehenden Ansprüche, wobei das Mehl ausgewählt ist aus der Gruppe bestehend aus Weizenmehl, Maismehl, Roggenmehl, Gerstenmehl, Hafermehl, Reismehl, Hirsemehl und einer Kombination davon.

**5.** Verfahren nach einem der vorhergehenden Ansprüche, wobei die Gamma-Glutamyltranspeptidase in einer Menge im Bereich von 0,01-100 mg Enzymprotein pro kg Mehl zugesetzt wird.

**6.** Verfahren nach einem der vorangehenden Ansprüche, wobei zusätzlich Glutathion zugesetzt wird.

**7.** Verfahren nach einem der vorhergehenden Ansprüche, wobei der Teig zudem ein oder mehrere Enzyme enthält, die ausgewählt sind aus der Gruppe bestehend aus Amylase, maltogener Amylase, Beta-Amylase, Aminopeptidase, Carboxypeptidase, Katalase, zellulytischem Enzym, Chitinase, Cutinase, Cyclodextrin-Glykosyltransferase, Deoxyribonuklease, Esterase, Glucan-1,4-alpha-Maltotetrahydrolase, Glucanase, Galaktanase, Alpha-Galaktosidase, Beta-Galaktosidase, Glukoamylase, Glukoseoxidase, Alpha-Glukosidase, Beta-Glukosidase, Haloperoxidase, hemizellulytischem Enzym, Invertase, Laccase, Lipase, Mannanase, Mannosidase, Oxidase, pektinolytischen Enzymen, Peptidoglutaminase, Peroxidase, Phospholipase, Phytase, Polyphenoloxidase, proteolytischem Enzym, Ribonuklease, Transglutaminase und Xylanase.

**8.** Verfahren nach einem der vorhergehenden Ansprüche, wobei das Fladenbrot ausgewählt ist aus der Gruppe bestehend aus Tortillas, Pitabrot, arabischem Brot, indischem Fladenbrot, weizen- und glutenfreiem Fladenbrot.

**9.** Verwendung einer Gamma-Glutamyltranspeptidase (E.C.2.3.2.2) zur Erhöhung der Dehnbarkeit eines Teigs, wobei die Gamma-Glutamyltranspeptidase mindestens 60 % Identität mit SEQ ID NO:1 aufweist, wobei der Teig eine Dehnbarkeit aufweist, die im Vergleich zu der Dehnbarkeit eines Teigs, der unter den gleichen Bedingungen, aber ohne Behandlung mit der Gamma-Glutamyltranspeptidase hergestellt wird, erhöht ist.

**Revendications**

**1.** Procédé pour l'augmentation de l'extensibilité d'une pâte comprenant

a) l'ajout d'une gamma-glutamyltranspeptidase (E.C.2.3.2.2) à de la farine ou à une pâte comprenant une farine, la gamma-glutamyltranspeptidase possédant au moins 60 % d'identité avec la SEQ ID N° 1 ;et

b) la préparation de la pâte,

la pâte possédant une extensibilité qui est augmentée par comparaison avec l'extensibilité d'une pâte qui est préparée dans les mêmes conditions, mais sans traitement avec la gamma-glutamyltranspeptidase.

2. Procédé selon la revendication 1, une pâte aplatie étant produite à partir de la pâte.

3. Procédé selon l'une quelconque des revendications précédentes, la pâte étant transformée en un produit comestible choisi dans le groupe constitué par un pain, un pain plat, des biscuits salés, des pâtes, des nouilles, des produits de boulangerie à pâte feuilletée, des biscuits, des baguettes, des hamburgers, et des pizzas.

4. Procédé selon l'une quelconque des revendications précédentes, la farine étant choisie dans le groupe constitué par de la farine de blé, de la farine de maïs, de la farine de seigle, de la farine d'orge, de la farine d'avoine, de la farine de riz, de la farine de sorgho, et une combinaison correspondante.

5. Procédé selon l'une quelconque des revendications précédentes, la gamma-glutamyltranspeptidase étant ajoutée en une quantité dans la plage de 0,01 à 100 mg de protéine enzymatique par kg de farine.

6. Procédé selon l'une quelconque des revendications précédentes, du glutathion étant de plus ajouté.

7. Procédé selon l'une quelconque des revendications précédentes, la pâte comprenant en outre une ou plusieurs enzymes choisies dans le groupe constitué par une amylase, une amylase maltogène, une bêta-amylase, une aminopeptidase, une carboxypeptidase, une catalase, une enzyme cellulytique, une chitinase, une cutinase, une cyclodextrine glycosyltransférase, une désoxyribonucléase, une estérase, une glucane 1,4-alpha-maltotétrahydrolase, une glucanase, une galactanase, une alpha-galactosidase, une bêta-galactosidase, une glucoamylase, une glucose oxydase, une alpha-glucosidase, une bêta-glucosidase, une halogénoperoxydase, une enzyme hémicellulytique, une invertase, une laccase, une lipase, une mannanase, une mannosidase, une oxydase, des enzymes pectinolytiques, une peptidoglutaminase, une peroxydase, une phospholipase, une phytase, une polyphénoloxydase, une enzyme protéolytique, une ribonucléase, une transglutaminase, et une xylanase.

8. Procédé selon l'une quelconque des revendications précédentes, le pain plat étant choisi dans le groupe constitué par des tortillas, un pain pita, un pain arabe, un pain plat indien, un pain plat exempt de blé et de gluten.

9. Utilisation d'une gamma-glutamyltranspeptidase (E.C.2.3.2.2) pour l'augmentation de l'extensibilité d'une pâte, la gamma-glutamyltranspeptidase possédant au moins 60 % d'identité avec la SEQ ID N° 1, la pâte possédant une extensibilité qui est augmentée par comparaison avec l'extensibilité d'une pâte qui est préparée dans les mêmes conditions, mais sans traitement avec la gamma-glutamyltranspeptidase.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013092731 A
- JP 4281787 B
- JP 2065777 A
- WO 02077009 A
- WO 1999043794 A
- WO 2006032281 A
- WO 2008148845 A
- WO 1999050399 A
- WO 2004111217 A
- WO 2005003339 A
- WO 8402921 A
- WO 9826057 A
- WO 0032758 A
- US 4106991 A
- US 4661452 A
- US 8431382 B
- EP 1766002 B1
- US 20140106457 A
- US 6066473 A

### Non-patent literature cited in the description

- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **TATE ; MEISTER.** *Mol. Cell. Biochem.,* 1981, vol. 39, 357-368
- **H. NEURATH ; R.L. HILL.** The Proteins. Academic Press, 1979
- **BOEL et al.** *EMBO J.,* 1984, vol. 3 (5), 1097-1102
- *Agric. Biol. Chem.,* 1991, vol. 55 (4), 941-949